# EUROPÄISCHE PATENTANMELDUNG

(11) **EP 1 000 624 A1**
(43) Veröffentlichungstag der Anmeldung: **17.05.2000**
(21) Anmeldenummer: 99119139.6
(22) Anmeldetag: 05.10.1999
(51) Int. Cl.: A61K 31/665

(54) **Verwendung von DOP als Antioxidations-, Alterungsschutz- und Arzneimittel**

(30) Priorität: 13.10.1998 DE 19847137
(71) Anmelder: Schill & Seilacher GmbH & Co., D-22113 Hamburg (DE)
(72) Erfinder: Die Erfindernennung liegt noch nicht vor
(74) Vertreter: Bunke, Holger, Dr.rer.nat. Dipl.-Chem.

(57) **Zusammenfassung**

Es wird die Verwendung der bekannten Verbindung 9,10-Dihydro-9-oxa-10-phosphaphenanthren-10-oxid ("DOP") und von DOP enthaltenden Zusammensetzungen als Antioxidationsmittel, als Alterungsschutzmittel und als antioxidativ wirksames Arzneimittel vorgeschlagen. DOP und DOP enthaltende Zusammensetzungen sind nicht toxisch, als Antioxidantien aber wirksamer als Vitamin E.

DOP kann als Additiv für Polyalkylenglykol, Fett, Öl, Hydraulik-Fluid, Schmiermittel, natürlichen oder synthetischen Gummi, Elastomer, Kunststoff, Nahrungsmittel, Futtermittel, Arzneimittel oder Kosmetikum zugegeben werden.

## Beschreibung

Die Erfindung betrifft die Verwendung von DOP und DOP enthaltenden Zusammensetzungen als Antioxidationsmittel, Alterungsschutzmittel und Arzneimittel.

Als "DOP" wird die bekannte Verbindung (6H)-Dibenz-(c,e)(1,2)-oxaphosphorin-6-on verstanden, die auch als 9,10-Dihydro-9-oxa-10-phosphaphenanthren-10-oxid bezeichnet werden kann und die in tautomerem Gleichgewicht mit der ebenfalls bekannten Verbindung 6-Hydroxy-(6H)-dibenz-(c,e)(1,2)-oxaphosphorin steht. In handelsüblichen Produkten liegt DOP häufig im Gemisch mit 2'-Hydroxydiphenyl-2-phosphinsäure vor, welche aus DOP durch hydrolytische Ringspaltung nach folgendem Reaktionsschema entsteht:

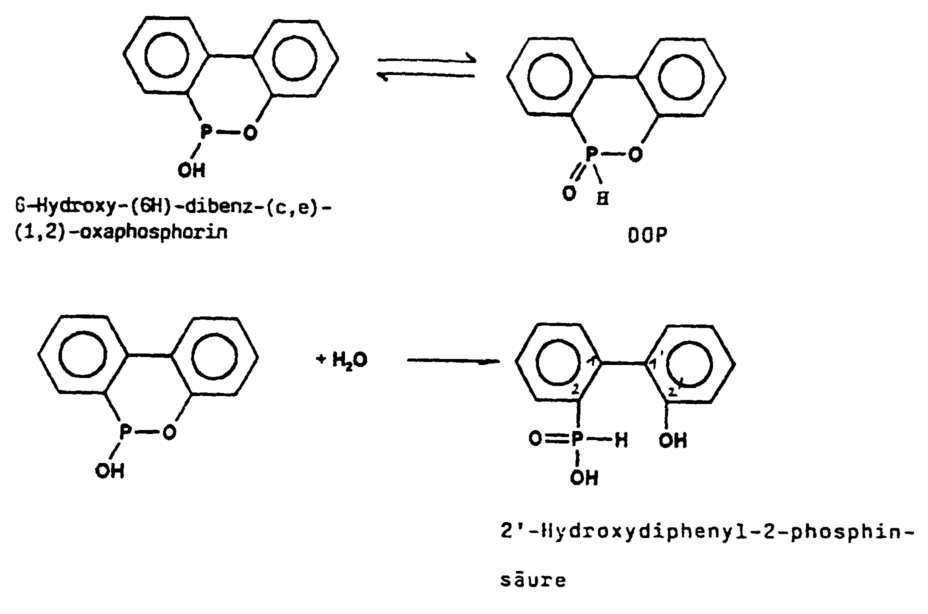

Ein Verfahren zur Herstellung eines solchen DOP-haltigen Gemisches ist beispielsweise aus DE 195 22 876 C1 bekannt.

Bisher wurden DOP und seine Derivate als Flammschutzmittel, insbesondere als copolykondensierbare Flammschutzmittel für Polyesterfasern verwendet; diese Verwendung ist beispielsweise in DE 26 46 218 C2 beschrieben.

Antioxidationsmittel (Antioxidantien) und Alterungsschutzmittel sind Additive, die in nahezu sämtlichen Bereichen der chemischen und pharmazeutischen Industrie sowie der Nahrungs- und Futtermittelindustrie eingesetzt werden, um Chemikalien oder daraus hergestellte Erzeugnisse, Naturstoffe, Kunststoffe, Nahrungs- und Genußmittel, Arzneimittel und Kosmetika vor Zersetzung, oxidativem Abbau, vorzeitiger Alterung und Autoxidation zu schützen. Obwohl bereits sehr viele verschiedene Antioxidantien bekannt sind (vgl. zum Beispiel die Übersicht in Ullmanns Encyklopädie der Technischen Chemie, 4. Auflage, Band 8, Seite 25 bis 42 (1974), besteht immer noch ein Bedürfnis, dem Fachmann weitere Antioxidations- und Alterungsschutzmittel an die Hand zu geben, insbesondere solche, die weniger toxisch und für Menschen, Tiere und Umwelt besser verträglich sind als die bisher bekanntgewordenen Antioxidantien.

Der Chemismus der meisten Antioxidatien ist sehr komplex und vielschichtig und daher im Detail meist ungeklärt. Bekannt ist lediglich, daß die meisten Antioxidantien Radikalfänger sind und deshalb zum Kettenabbruch radikalisch ablaufender Reaktionsketten führen. Bekannt ist ferner, daß viele Antioxidantien Peroxide zersetzen und dabei selbst oxidiert werden. Bei diesen Reaktionen spielen aber viele weitere Faktoren eine Rolle, vor allem die Einwirkung von Licht und UV-Strahlung, die Anwesenheit katalytisch wirksamer Metallionen und vieles mehr. Aus diesem Grunde gibt es für jedes zu schützende Produkt meist nur eine kleine Zahl von geeigneten Antioxidationsmitteln bzw. Alterungsschutzmitteln, deren Eigenschaften, wie zum Beispiel ihre eigene Toxizität und Verträglichkeit, und Wirkungen einen unter den gegebenen Umständen optimalen Kompromiß darstellen.

Im Zuge der vorliegenden Erfindung wurde nun überraschenderweise gefunden, daß DOP und DOP enthaltende Zusammensetzungen als Antioxidationsmittel und als Alterungsschutzmittel hervorragend wirksam sind, gleichzeitig aber eine extrem geringe Toxizität aufweisen und weder augenreizend noch hautreizend sind.

Gegenstand der Erfindung ist deshalb die Verwendung von DOP und DOP enthaltenden Zusammensetzungen als Antioxidationsmittel und als Alterungsschutzmittel.

Besonders bevorzugt ist die Verwendung von DOP und DOP enthaltenden Zusammensetzungen als schützendes Additiv, das einem Polyalkylenglykol, Fett, Öl, Hydraulik-Fluid, Schmiermittel, natürlichen oder synthetischen Gummi, Elastomer, Kunststoff, Nahrungsmittel, Futtermittel, Arzneimittel oder Kosmetikum zugegeben wird.

Vorzugsweise wird das Additiv in einer Menge von 0,01 bis 10 Gew.-% zugegeben, besonders bevorzugt in einer Menge von 0,5 bis 2,5 Gew.-%.

DOP und DOP enthaltende Zusammensetzungen können aber nicht nur Arzneimitteln als schützendes Additiv zugegeben werden, sie stellen aufgrund ihrer geringen Toxizität und ihrer Bioverträglichkeit auch selbst Arzneimittel dar, die *in vivo* antioxidativ wirksam sind.

Gegenstand der Erfindung sind deshalb auch Arzneimittel, die eine antioxidativ wirksame Menge an DOP oder einer DOP enthaltenden Zusammensetzung als Wirkstoff enthalten, gegebenenfalls zusammen mit pharmakologisch verträglichen Hilfs- und Trägerstoffen.

DOP und DOP enthaltende Zusammensetzungen sind somit in gleicher Weise verwendbar wie Vitamin E (ein Gemisch aus Tocopherolen), das bekanntlich sowohl als Arzneimittel, als auch zur Vitaminisierung von Nahrungs- und Futtermitteln, als auch als Antioxidans und Alterungsschutzmittel für Fette, Öle, Kosmetika und für Arzneimittel seit vielen Jahren erfolgreich eingesetzt wird (vgl. Ullmanns Enzyklopädie a.a.O., Seite 30).

Die akute Toxizität von DOP wurde peroral an der Ratte bestimmt; die letale Dosis, bei der 50% der Versuchstiere verenden (LD₅₀), beträgt mehr als 2000 mg/kg.

Um festzustellen, ob DOP Reizungen der Augen oder der Haut hervorrufen kann, wurde am Kaninchen ein Augenreiztest und ein Hautreiztest durchgeführt, beide gemäß der Verordnung der EU-Kommission Nr. 93/21/EEC vom 27.04.1993. Die Auswirkungen von DOP auf Kornea, Iris und Konjunktiva waren nach 24, 48 und 72 Stunden so gering, daß die Substanz als nicht augenreizend im Sinne der genannten Verordnung eingestuft werden konnte. Der Hautreiztest erbrachte weder eine Erythembildung noch eine Oedembildung nach 24, 48 und 72 Stunden, so daß DOP als nicht hautreizend im Sinne der Verordnung gilt.

Um die antioxidative Wirksamkeit von DOP im Vergleich mit bekannten Antioxidantien festzustellen, wurde der "Rotating Bomb Test" (RBOT) gemäß der ASTM-Norm D 2272-85 durchgeführt. Bei diesem Test wird ein Hydraulik-Fluid, ein Öl oder dgl. zusammen mit Wasser und einer Kupferdrahtspirale in einem rotierenden Druckbehälter mit Sauerstoff unter einem Druck von 620 kPa (90 psi) bei 150 °C beaufschlagt und in Schräglage (30° zur Horizontalen) mit 100 Umdrehungen pro Minute rotiert. Die Zeit (in Minuten), die erforderlich ist, um einen bestimmten Druckabfall infolge des verbrauchten Sauerstoffs hervorzurufen, stellt ein Maß für die Oxidationsbeständigkeit des untersuchten Öls bzw. der untersuchten Flüssigkeit dar.

Im vorliegenden Falle wurde die Wirkung verschiedener Additive auf Emkarate 1550 ® ein Polyolester der Firma ICI, untersucht. Als bekanntes Antioxidans wurde zum Vergleich Irganox ® E 201 (Vitamin E), der Firma Ciba Specialty Chemicals Inc. verwendet, und zwar in einer Konzentration von 1,0 Gew.-%, bezogen auf das Gewicht des Polyolesters. Eine handelsübliche, DOP enthaltende Zusammensetzung, nämlich Ukanol ® DOP der Schill & Seilacher GmbH & Co., wurde als bevorzugtes Beispiel für die erfindungsgemäße Verwendung getestet.

Wie sich aus der nachfolgenden Tabelle ergibt, betrug die Oxidationsbeständigkeit ohne jedes Additiv 50 Minuten, mit Zusatz von 1 Gew.-% Vitamin E als Antioxidans 98 Minuten, bei erfindungsgemäßer Verwendung von 0,5 Gew.-% Ukanol DOP aber bereits 109 Minuten und schließlich bei erfindungsgemäßer Verwendung von 1,0 Gew.-% Ukanol 134 Minuten:

**Tabelle**

| Additiv (Antioxidans) | Konz. (Gew.-%) | Oxidationsbeständigkeit [min] |
|---|---|---|
| - | - | 50 |
| Irganox E 201 (Vit. E) | 1,0 | 98 |
| Ukanol DOP | 0,5 | 109 |
| Ukanol DOP | 1,0 | 134 |

Diese Ergebnisse zeigen, daß DOP und DOP enthaltende Zusammensetzungen ebensowenig toxisch wie die Tocopherole des Vitamins E sind, gleichzeitig weder augenreizend noch hautreizend sind, aber eine erheblich bessere antioxidative Wirkung haben als Vitamin E. Deshalb sind DOP und DOP enthaltende Zusammensetzungen nicht nur als Antioxidationsmittel für technische Produkte und Chemikalien verwendbar, sondern auch als Antioxidationsmittel für Nahrungs- und Futtermittel, für Arzneimittel und Kosmetika. Außerdem sind DOP und DOP enthaltende Zusammensetzungen auch selbst als Arzneimittel verwendbar, vergleichbar mit Vitamin E.

## Patentansprüche

1. Verwendung von DOP (9,10-Dihydro-9-oxa-10-phosphaphenanthren-10-oxid) und DOP enthaltenden Zusammensetzungen als Antioxidationsmittel.

2. Verwendung von DOP (9,10-Dihydro-9-oxa-10-phosphaphenanthren-10-oxid) und DOP enthaltenden Zusammensetzungen als Alterungsschutzmittel.

3. Verwendung nach Anspruch 1 oder 2, dadurch gekennzeichnet, daß das DOP oder die DOP enthaltende Zusammensetzung einem Polyalkylenglykol, Fett, Öl, Hydraulik-Fluid, Schmiermittel, natürlichen oder synthetischen Gummi, Elastomer, Kunststoff, Nahrungsmittel, Futtermittel, Arzneimittel oder Kosmetikum als Additiv zugegeben wird.

4. Verwendung nach Anspruch 3, dadurch gekennzeichnet, daß das Additiv in einer Menge von 0,01 bis 10 Gew.-% zugegeben wird.

5. Verwendung nach Anspruch 4, dadurch gekennzeichnet, daß das Additiv in einer Menge von 0,5 bis 2,5 Gew.-% zugegeben wird.

6. Arzneimittel, enthaltend eine antioxidativ wirksame Menge an DOP (9,10-Dihydro-9-oxa-10-phosphaphenanthren-10-oxid) oder einer DOP enthaltenden Zusammensetzung als Wirkstoff, gegebenenfalls zusammen mit pharmakologisch verträglichen Hilfs- und Trägerstoffen.
